# EUROPEAN PATENT APPLICATION

(11) **EP 4 064 290 A2**
(43) Date of publication of application: **28.09.2022**
(21) Application number: 20890564.6
(22) Date of filing: 28.10.2020
(51) Int. Cl.: G16H 40/20, G16H 70/00, G16H 20/10, G16H 40/40, G06F 16/2457, G06F 16/2455, G06F 16/33, G06F 16/93

(54) **SYSTEM AND METHOD FOR PROVIDING MEDICAL EQUIPMENT AND MEDICINE INFORMATION**

(30) Priority: 18.11.2019 KR 20190147403
(71) Applicant: Medalls Co., Ltd., Gangnam-gu, Seoul 06235 (KR)
(72) Inventor: HAN, Sang Wook, Seongnam-si, Gyeonggi-do 13536 (KR); YOO, Woo Kyoung, Gunpo-si, Gyeonggi-do 15821 (KR)
(74) Representative: FRKelly
(86) International application number: PCT/KR2020/014760
(87) International publication number: WO 2021/101106

(57) **Abstract**

The present invention relates to a method for providing medical equipment and medicine information, and comprises: a medical paper database that provides medical papers; a medical equipment and medicine database that provides medical equipment and medicine information; and a service server that finds, from the medical equipment and medicine database, information about medical equipment and medicine and posts the found medical equipment and medicine on a network, and also periodically searches the medical paper database for the papers in which the posted medical equipment and medicine have been described to provide a user terminal with paper information and links.

## Description

### BACKGROUND

### 1. Field of the Invention

The present invention relates to a system and method for providing medical device and medicine information, and more particularly, to a system and method for providing accurate information on medical devices and medicines to doctors or nurses.

### 2. Discussion of Related Art

These days, systems and methods for providing services that meet various user needs by utilizing big data are actively under development.

In particular, with the development of computer networks, such as the Internet, individuals may easily post various standardized or non-standardized documents on a network. The standardized documents may be documents complying with standard international protocols or articles which do not necessarily comply with standard protocols but are in specific forms.

The non-standardized documents may be documents which are not in specific forms but are intended to provide specific information in the form of news, advertisements, or blogs.

Such posted documents may be used as indicators of a technical development direction, a market trend, etc. in addition to the concentration of information as well as the construction of big data. Therefore, a technology for classifying and collecting characteristics of posted documents is very important in the current data processing field.

Meanwhile, documents posted on networks may provide professional and accurate information but also include incorrect information or knowledge.

For example, documents for advertisement and promotion of a product often describe the actual function with exaggeration, and records of using products or medicines may provide different results from actual performance and effects depending on the writer's personal opinion.

Generally, it is difficult to distinguish such incorrect information and knowledge from correct information and knowledge.

In particular, most information and knowledge about expert medical devices or medicines including reagents is provided by manufacturers or distributor of medical devices or medicines. Accordingly, such information and knowledge is advertising information and knowledge rather than accurate information and knowledge, thus requiring verification.

### RELATED ART DOCUMENTS

### Patent Documents

Korean Patent 10-0526438 (System and method for providing journal information, filed Oct 28, 2005)

### SUMMARY

The present invention is directed to providing a system and method for providing medical device and medicine information which may provide highly-reliable expert information on medical devices and medicines.

More specifically, the present invention is directed to providing a system and method for searching for articles including usage, experiments, and test results regarding medical devices and medicines, extracting information on medical devices and medicines from found articles, and providing the extracted information to users.

According to an aspect of the present invention, there is provided a system for providing medical device and medicine information, the system including a medical articles database configured to provide medical articles, a medical device and medicine database configured to provide medical device and medicine information, and a service server configured to search the medical device and medicine database for information on a medical device or medicine, post found information on a network, periodically search the medical articles database for articles written on the posted medical device or medicine, and provide article information and an article link to a user terminal.

The service server may perform a keyword search in the medical articles database to search for articles and reset a keyword to perform a keyword search again when a number of articles which are search results deviates from a set range.

The service server may set rankings depending on numbers of times that the keyword is found in the articles and extract text of high-ranking articles, and the system may further include an artificial intelligence server configured to learn the extracted text and provide utilization example information of the medical device or medicine to the service server.

According to another aspect of the present invention, there is provided a method of providing medical device and medicine information, the method including (a) extracting, by a service server, a keyword from product information of a medical device or a medicine, (b) searching, by the service server, a medical articles database for articles written on the product using the extracted keyword, (c) determining, by the service server, whether a number of found articles is within a set range, (d) updating, by the service server, the keyword when it is determined in operation (c) that the number of found articles is not within the set range and performing operation (b) again, and (e) generating, by the service server, a link of a search result page and posting the link together with the product information when it is determined in operation (c) that the number of found articles is within the set range.

The method may further include, when it is determined in operation (c) that the number of found articles is within the set range, ranking, by the service server, the found articles depending on a number of times that the keyword is found, extracting text from a set number of high-ranking articles, learning, by an artificial intelligence server, the extracted text and extracting and providing a utilization example of the product to the service server, and posting, by the service server, the utilization example together with the product information on a webpage.

The extracting of the text may include extracting text of a paragraph including the keyword or a set number of paragraphs among which the paragraph including the keyword is centered.

The utilization example may be extracted with a measure centered therein.

### BRIEF DESCRIPTION OF THE DRAWINGS

The above and other objects, features and advantages of the present invention will become more apparent to those of ordinary skill in the art by describing exemplary embodiments thereof in detail with reference to the accompanying drawings, in which:
FIG 1 is a block diagram of a system for providing medical device and medicine information according to an exemplary embodiment of the present invention;
FIG. 2 is an exemplary diagram of a service page provided by a service server;
FIG 3 is a flowchart illustrating a method of providing medical device and medicine information according to an exemplary embodiment of the present invention;
FIG. 4 is a block diagram of a system for providing medical device and medicine information according to another exemplary embodiment of the present invention; and
FIG 5 is a flowchart illustrating a method of providing medical device and medicine information according to another exemplary embodiment of the present invention.

### DETAILED DESCRIPTION

Hereinafter, a system and method for providing medical device and medicine information according to the present invention will be described in detail with reference to the accompanying drawings.

Exemplary embodiments of the present invention are provided to fully convey the present invention to those of ordinary skill in the art. The exemplary embodiments to be described below may be modified into various forms, and the scope of the present invention is not limited to the following embodiments. Rather, the embodiments are provided to make the present invention thorough and complete and convey the spirit of the present invention to those of ordinary skill in the art.

Terms used herein are used to describe specific embodiments and not intended to limit the present invention. As used herein, the singular forms may include the plural forms as well unless the context clearly indicates otherwise. Also, as used herein, the terms "comprise" and/or "comprising" specify the presence of stated shapes, numerals, steps, operations, members, elements, and/or a group thereof and do not preclude the presence or addition of one or more other shapes, numerals, steps, operations, members, elements, and/or a group thereof. As used herein, the term "and/or" includes each of listed items and all combinations of one or more thereof.

In this specification, terms such as "first," "second," etc. are used to describe various members, regions, and/or portions. However, it is obvious that the members, parts, regions, layers, and/or portions are not limited to these terms. The terms do not mean a particular order, up and down, or superiority and are used only for distinguishing one member, region, or portion from another member, region, or portion. Accordingly, a first member, region, or portion to be described below may also refer to a second member, region, or portion without departing from the teaching of the present invention.

Hereinafter, embodiments of the present invention will be described with reference to drawings which schematically show the embodiments of the present invention. Shapes illustrated in the drawings may be varied according to manufacturing techniques and/or tolerances. Accordingly, the embodiments are not construed as being limited to specific shapes of illustrated regions and include, for example, shape changes caused in manufacturing processes.

According to the present invention, articles posted on a network are searched for articles written on information on medical devices and medicines, and found articles are matched to the medical devices and the medicines such that users are provided with highly-reliable expert information. Further, information on the usage, conditions of use, and results of use of medical devices and medicines is extracted, processed, and provided. The present invention is performed by a device including a processor for processing, a database for storage, a display for a user's check, an input device for a user input, etc.

In the present invention, the terms "server" and "database" are used, which mean physically-specified calculation device and storage device, respectively. The server may be a device which may perform various calculations including a general computer. The present invention may employ any database in which documents tagged with keywords are stored in regions indexable according to a classification system of the server regardless of the storage method or shape.

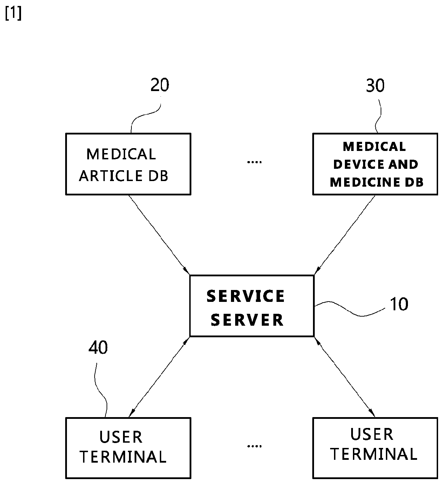

FIG. 1 is a block diagram of a system for providing medical device and medicine information according to an exemplary embodiment of the present invention.

Referring to FIG. 1, the system for providing medical device and medicine information according to the exemplary embodiment of the present invention includes a medical articles database 20 which provides medical articles, a medical device and medicine database 30 which provides medical device and medicine information, and a service server 10 which searches the medical device and medicine database 30 for information on a medical device or medicine to post found information on a network and also periodically searches the medical articles database 20 for articles written on the posted medical device or medicine to provide article information and an article link to user terminals 40.

The above configuration and operations of the system for providing medical device and medicine information according to the exemplary embodiment of the present invention will be described below.

First, the service server 10 is a server that provides a web service to the user terminals 40 connected thereto.

Also, the medical articles database 20 provides a search service through a server for providing medical information. The medical articles database 20 may employ the search system of Medisurf (http://www.medisurf.com) which provides a medical expertise service or may be a database provided by a server which provides another medical search service.

Also, the medical articles database 20 may be a database storing article information of the medical field in a library.

The medical device and medicine database 30 may be a database provided by a manufacturer that manufactures a medical device or medicine or a distributor.

The service server 10 extracts information on medical devices and medicines from the medical device and medicine database 30, classifies the information, and posts the classification results in a web.

Here, a separate page may be implemented for each medical device or medicine, or the medical devices and medicines may be classified by type, and a table may be generated for each classification such that a list may be posted.

The list may be linked to a product introduction page of a manufacturer or distributor of each medical device or medicine.

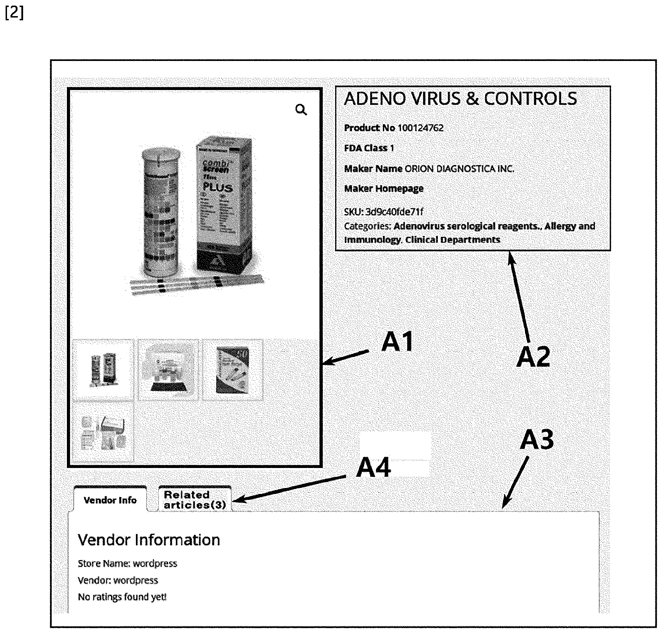

FIG. 2 is an exemplary diagram of a service screen of the service server 10.

Referring to FIG. 2, the service server 10 separately posts a medical device or medicine on a webpage. The webpage may include a photo area A1 in which a photo of a product is displayed, an information area A2 in which a general description of the product is displayed, and a sale information area A3 in which sale information is displayed.

Also, a related article link area A4 may be provided in a portion of the service screen of the service server 10.

When a user who accesses the service server 10 using the user terminal 40 selects the related article link area A4, a new screen for checking article information found by the service server 10 may be displayed.

The service server 10 searches the medical articles database 20 using information retrieved from the medical device and medicine database 30 and generates a link to an article written on the product, thereby enabling the user to check a utilization example of the medical device or medicine.

In this case, a search keyword for the medical articles database 20 may be a model name, a product name, a company name, a product type, etc. of the medical device or medicine.

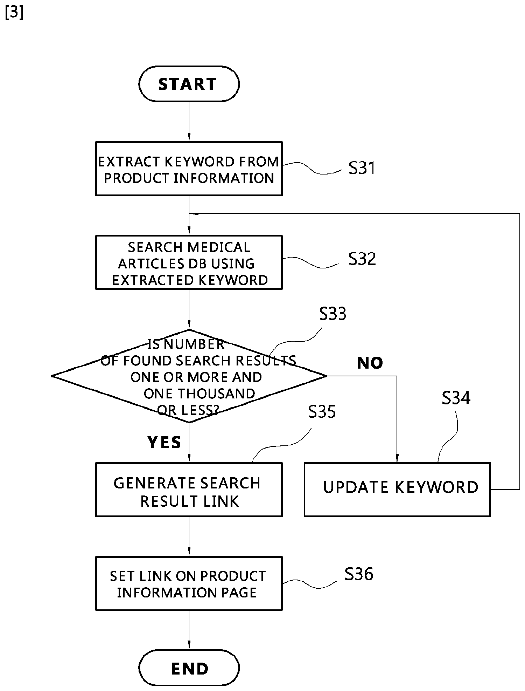

FIG. 3 is a flowchart illustrating a method of providing medical device and medicine information according to an exemplary embodiment of the present invention.

Referring to FIG. 3, the method of providing medical device and medicine information according to the present invention includes an operation S31 of extracting a keyword from product information, an operation S32 of searching the medical articles database 20 using the extracted keyword, an operation S33 of determining whether the number of found medical articles is within a set range, an operation S34 of updating the keyword and reperforming the operation S32 when it is determined in the operation S33 that the number of found medical articles is not within the set range, an operation S35 of generating a link to a search result page when it is determined in the operation S33 that the number of found medical articles is within the set range, and an operation S36 of setting the link on a product information page from which the keyword has been extracted.

The above method of providing medical device and medicine information according to the present invention will be described in further detail below.

First, as described above, the service server 10 checks the medical device and medicine database 30 to determine whether there is information on new medical devices or medicines.

The medical device and medicine database 30 may be provided by a server of a manufacturer or distributor, and the service server 10 may periodically search the medical device and medicine database 30 to retrieve newly registered information of the medical device and medicine database 30.

In the present invention, the service server 10 is illustrated and described to check the medical device and medicine database 30. However, the manufacturer or distributor of a medical device or medicine may request the service server 10 to register a new medical device or medicine therein.

When there is a new medical device or medicine, the service server 10 generates a new page and enters information on the medical device or medicine in the new page.

An example of a screen displayed in this case has been described above with reference to FIG. 2.

As described in the operation S31, to implement the related article link area A4, the service server 10 extracts a keyword from product information to be posted.

Here, the keyword may be set in order of a model name, a product name, a company name, a product type, and a classification of the medical device or medicine.

Subsequently, as described in the operation S32, the medical articles database 20 is searched using the extracted keyword.

In other words, the medical articles database 20 is searched to determine whether there is an article including a medical device or medicine corresponding to the keyword in the content among medical articles.

Subsequently, in the operation S33, it is determined whether the number of medical articles found in the operation S32 is within the set range.

The range of the number of found medical articles may be set to 1 or more and 1000 or less or the like. In other words, when no medical article is found or the number of found medical articles exceeds 1000, the service server 10 may determine that it is necessary to reset the keyword.

The range of the number of found medical articles may be arbitrarily set. A minimum value is set to 1 in consideration of a case in which no article is found. On the other hand, reliability may be degraded when the number of found articles is less than 10. Accordingly, a minimum value is set to 10 to reset the keyword when less than 10 articles are found.

Likewise, a maximum value may be set to 100 or less or the like. When an excessive number of medical articles are found, other processing times to be described below are delayed, and too much information is provided to a user, which confuses the user. To prevent these, it is allowed to find an appropriate number of articles by resetting the keyword.

Subsequently, as described in the operation S34, when it is determined in the operation S33 that the number of medical articles is not within the set range, the keyword is updated, and then in the operation S32, the medical articles database 20 is searched again using the updated keyword.

The keyword may be updated in various ways. The number of found medical articles may be adjusted by calculating the logical sum or logical product of keywords.

When it is determined in the operation S33 that the number of medical articles is within the set range, a link of a page in which articles that are search results are displayed as described is generated in the operation S35.

Subsequently, as described in the operation S36, the generated link is set to allow movement to a page in which the found articles may be checked when the above-described related article link area A4 is selected.

With this process, the service server 10 can provide articles written on a medical device or medicine in a page which provides the corresponding medical device or medicine information so that users can easily check the articles.

Users of the user terminals 40 may be doctors or nurses who actually use medical devices or medicines and can check the usage or effect of a specific medical device or medicine in advance by reading articles.

In other words, the present invention provides highly reliable information on medical devices or medicines, thereby providing help necessary for users to use medical devices or medicines.

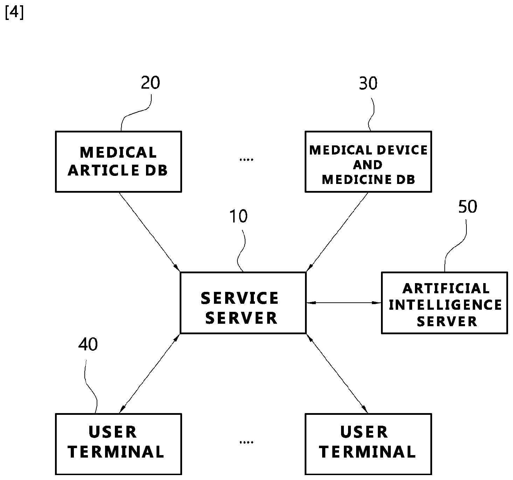

FIG. 4 is a block diagram of a system for providing medical device and medicine information according to another exemplary embodiment of the present invention.

Referring to FIG. 4, the system for providing medical device and medicine information according to the other exemplary embodiment of the present invention includes a medical articles database 20 which provides medical articles, a medical device and medicine database 30 which provides medical device and medicine information, a service server 10 which searches the medical device and medicine database 30 for information on a medical device or medicine to post found information on a network, periodically searches the medical articles database 20 for articles written on the posted medical device or medicine to provide article information and an article link to user terminals 40, and also extracts text from an article to display and provide a utilization example of the medical device or medicine to the user terminals 40, and an artificial intelligence server 50 which learns the text extracted from the article and reselects and provides an area suitable for the utilization example to the service server 10.

The above configuration and operations of the system for providing medical device and medicine information according to the other exemplary embodiment of the present invention will be described in further detail below.

First, the service server 10 is a server that provides a web service to the user terminals 40 connected thereto. As described above, the service server 10 searches the medical device and medicine database 30 or posts information on new medical devices and medicines on a webpage on the basis of provided information, thereby providing the information to the user terminals 40.

Also, the service server 10 determines a keyword in the medical device and medicine information and searches the medical articles database 20 for medical articles including the determined keyword. Then, the service server 10 posts a link so that found articles may be easily checked through the user terminals 40. This is the same as described above.

In addition, the service server 10 may rank the found medical articles.

The service server 10 may check the frequencies of the search keyword in found medical articles and set rankings in decreasing order of frequency. Only a set number of rankings are determined to be valid.

For example, three high-ranking articles, five high-ranking articles, etc. may be set.

Further, the service server 10 may extract text from articles of valid rankings.

The extracted text may be all or a part of the text of a selected article.

In particular, when partial text is extracted, the extracted text may be a sentence including the keyword or a set number of paragraphs including a paragraph including the keyword and paragraphs before and after the paragraph.

For example, a total of 11 paragraphs may be set to include a paragraph including the keyword, five paragraphs before the paragraph, and five paragraphs after the paragraph.

The extracted text data may be provided to the artificial intelligence server 50, and the artificial intelligence server 50 may find text corresponding to a utilization example of a medical device or medicine through learning and provide the text to the service server 10.

Learning of the artificial intelligence server 50 may be set in a very variety of ways, but in the present invention, may be set as measure-based learning.

For example, in the case of a medicine, data of text including a measure representing a usage, such as mg, g, ml, cc, etc., may be extracted as a utilization example, and text corresponding to a utilization example may be accurately extracted through learning.

To this end, text corresponding to utilization examples is input in advance such that the artificial intelligence server 50 may learn the text.

As examples of training text, "inject 10 ml of a reagent," "after one hour has elapsed," "check status," "clinical results," etc. may be learned such that text corresponding to utilization examples of medical devices or medicines may be extracted.

The extracted text data of the utilization example may be provided to the service server 10, and the service server 10 may additionally generate the utilization example area which has been described above with reference to FIG. 2 and post the utilization example data in the page.

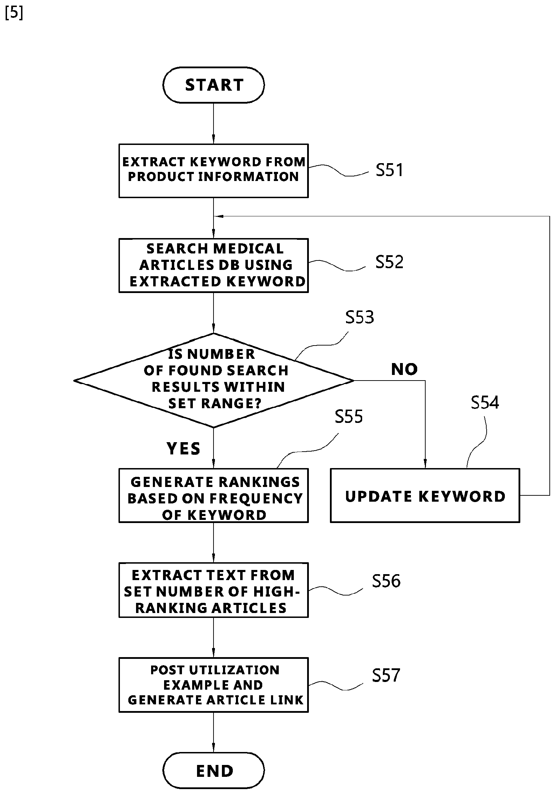

FIG. 5 is a flowchart illustrating a method of providing medical device and medicine information according to another exemplary embodiment of the present invention.

Referring to FIG. 5, the method of providing medical device and medicine information according to the present invention includes an operation S51 of extracting a keyword from product information, an operation S52 of searching the medical articles database 20 using the extracted keyword, an operation S53 of determining whether the number of found medical articles is within a set range, an operation S54 of updating the keyword and reperforming the operation S52 when it is determined in the operation S53 that the number of found medical articles is not within the set range, an operation S55 of generating rankings on the basis of the frequency of the keyword when it is determined in the operation S53 that the number of found medical articles is within the set range, an operation S56 of extracting text from the high-ranking articles, and an operation S57 of posting a utilization example, which is a result of training the artificial intelligence server 50 with the extracted text, and an article link.

In the above method of providing medical device and medicine information according to the other exemplary embodiment of the present invention, the service server 10 checks the medical device and medicine database 30 to determine whether there is information on new medical devices or medicines.

The medical device and medicine database 30 may be provided by a server of a manufacturer or distributor, and the service server 10 may periodically search the medical device and medicine database 30 to retrieve newly registered information of the medical device and medicine database 30.

In the present invention, the service server 10 is illustrated and described to check the medical device and medicine database 30. However, the manufacturer or distributor of a medical device or medicine may request the service server 10 to register a new medical device or medicine therein.

When there is a new medical device or medicine, the service server 10 generates a new page and enters information on the medical device or medicine in the new page.

Subsequently, as described in the operation S51, the service server 10 extracts a keyword, which is set in order of a model name, a product name, a company name, a product type, and a classification of the medical device or medicine, from product information to be posted.

Subsequently, as described in the operation S52, the medical articles database 20 is searched using the extracted keyword.

Subsequently, in the operation S53, it is determined whether the number of medical articles found in the operation S52 is within the set range. Since this has been described in detail above with reference to FIG. 3, the description will be omitted.

Subsequently, as described in the operation S54, when it is determined in the operation S53 that the number of medical articles is not within the set range, the keyword is updated, and then in the operation S52, the medical articles database 20 is searched again using the updated keyword.

The keyword may be updated in various ways. The number of found medical articles may be adjusted by calculating the logical sum or logical product of keywords.

When it is determined in the operation S53 that the number of medical articles is within the set range, the articles which are search results are ranked as described in the operation S55.

The rankings are determined according to the frequency of the keyword entered.

For example, when the same keyword is repeatedly entered ten times in a first article, seven times in a second article, and twelve times in a third article, the articles are ranked in order of the third article, the first article, and the second article.

Subsequently, as described in the operation S56, text is extracted from the articles within set rankings among the medical articles of which the rankings have been determined.

The extracted text may be the entire text of the articles or may be a sentence including the search keyword or a set number of paragraphs including the paragraph including the keyword and paragraphs before and after the paragraph.

Subsequently, as described in the operation S57, the artificial intelligence server 50 learns the extracted text and extracts a utilization example of the medical device or medicine.

Learning of the artificial intelligence server 50 may be set in a very variety of ways, but in the present invention, may be set as measure-based learning.

For example, in the case of a medicine, data of text including a measure representing a usage, such as mg, g, ml, cc, etc., may be extracted as a utilization example, and text corresponding to a utilization example may be accurately extracted through learning.

To this end, text corresponding to utilization examples is input in advance such that the artificial intelligence server 50 may learn the text.

As examples of training text, "inject 10 ml of a reagent," "after one hour has elapsed," "check status," "clinical results," etc. may be learned such that text corresponding to utilization examples of medical devices or medicines may be extracted.

The extracted text data of the utilization example may be provided to the service server 10, and the service server 10 may additionally generate the utilization example area which has been described above with reference to FIG. 2 and post the utilization example data in the page.

With this process, the service server 10 can provide articles written on a medical device or medicine in a page which provides the corresponding medical device or medicine information so that users can easily check the articles. Also, the service server 10 posts a utilization example together. Accordingly, it is possible to easily check a utilization example or effect of a specific medical device or medicine in advance.

According to the present invention, a medical articles database for providing medical articles and a medical device and medicine database for providing medical device and medicine information are provided. The medical device and medicine database is searched for medical device or medicine information, and found medical device or medicine information is posted on a network. Also, the medical articles database is periodically searched for articles written on the posted medical device or medicine, and article information and links are provided. Accordingly, it is possible to provide expert medical knowledge.

It is apparent to those of ordinary skill in the art that the present invention is not limited to the exemplary embodiments and can be variously modified or altered without departing from the technical spirit of the present invention.

The present invention retrieves medical device and medicine information entered in medical articles on the basis of the nature laws to provide highly reliable device or medicine information and thus is applicable to industries.

## Claims

1. A system for providing medical device and medicine information, the system comprising:
a medical articles database configured to provide medical articles;
a medical device and medicine database configured to provide medical device and medicine information; and
a service server configured to search the medical device and medicine database for information on a medical device or medicine, post found information on a network, periodically search the medical articles database for articles written on the posted medical device or medicine, and provide article information and an article link to a user terminal.

2. The system of claim 1, wherein the service server performs a keyword search in the medical articles database to search for articles and resets a keyword to perform a keyword search again when a number of articles which are search results deviates from a set range.

3. The system of claim 2, wherein the service server ranks the articles depending on numbers of times that the keyword is found in the articles and extracts text of high-ranking articles,
further comprising an artificial intelligence server configured to learn the extracted text and provide utilization example information of the medical device or medicine to the service server.

4. A method of providing medical device and medicine information, the method comprising:
(a) extracting, by a service server, a keyword from product information of a medical device or a medicine;
(b) searching, by the service server, a medical articles database for articles written on the product using the extracted keyword;
(c) determining, by the service server, whether a number of found articles is within a set range;
(d) updating, by the service server, the keyword when it is determined in operation (c) that the number of found articles is not within the set range and performing operation (b) again; and
(e) generating, by the service server, a link of a search result page and posting the link together with the product information when it is determined in operation (c) that the number of found articles is within the set range.

5. The method of claim 4, further comprising, when it is determined in operation (c) that the number of found articles is within the set range:
ranking, by the service server, the found articles depending on a number of times that the keyword is found;
extracting text from a set number of high-ranking articles;
learning, by an artificial intelligence server, the extracted text and extracting and providing a utilization example of the product to the service server; and
posting, by the service server, the utilization example together with the product information on a webpage.

6. The method of claim 5, wherein the extracting of the text comprises extracting text of a paragraph including the keyword or a set number of paragraphs among which the paragraph including the keyword is centered.

7. The method of claim 5, wherein the utilization example is extracted with a measure centered therein.
